(19) Europäisches Patentamt
European Patent Office
Office européen des brevets

(11) Veröffentlichungsnummer: **0 479 177 A2**

(12) # EUROPÄISCHE PATENTANMELDUNG

(21) Anmeldenummer: **91116632.0**

(22) Anmeldetag: **30.09.91**

(51) Int. Cl.5: **C07D 213/81**, A61K 31/44

(30) Priorität: **01.10.90 DE 4031000**

(43) Veröffentlichungstag der Anmeldung: **08.04.92 Patentblatt 92/15**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Baader, Ekkehard, Dr.**
**Amselweg 14**
**W-6240 Königstein/Taunus(DE)**
Erfinder: **Bickel, Martin, Dr.**
**Mittelstedter Weg 3**
**W-6380 Bad Homburg(DE)**
Erfinder: **Günzler-Pukall, Volkmar, Dr.**
**Gross-Seelheimer Strasse 13**
**W-3550 Marburg(DE)**
Erfinder: **Volz, Manfred, Dr.**
**Steinmetzstrasse 11**
**W-6390 Usingen(DE)**

(54) **4- Oder 5-substituierte Pyridin-2-carbonsäuren, Verfahren zu deren Herstellung sowie deren Verwendung als Arzneimittel.**

(57) Die Erfindung betrifft in 4- oder 5-Stellung mit Aminogruppen substituierte Pyridin-2-carbonsäuren und ihre Verwendung als Arzneimittel. Insbesondere wird die Wirkung der Verbindungen zur Inhibierung der Prolin- und Lysinhydroxylase und zur Behandlung von Störungen der Biosynthese von Kollagen und kollagenähnlichen Stoffen beschrieben.

EP 0 479 177 A2

Verbindungen, die die Enzyme Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird Protein-gebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Es ist bekannt, daß die Inhibierung der Prolinhydroxylase durch bekannte Inhibitoren wie $\alpha,\alpha'$-Dipyridyl zu einer Hemmung der $C1_q$-Biosynthese von Makrophagen führt (W. Müller et al., FEBS Lett. 90 (1978), 218; Immunobiology 155 (1978), 47). Dadurch kommt es zu einem Ausfall des klassischen Weges der Komplementaktivierung. Inhibitoren der Prolinhydroxylase wirken daher auch als Immunsuppressiva, z.B. bei Immunkomplexkrankheiten.

Es ist bekannt, daß das Enzym Prolinhydroxylase durch Pyridin-2,4- und -2,5-dicarbonsäuren effektiv gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 248 (1987) 625-633).

In der DE-A 34 32 094 werden Pyridin-2,4- und -2,5-dicarbonsäurediester mit 1-6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Die DE-A 37 03 959, DE-A 37 03 962 und DE-A 37 03 963 beschreiben in allgemeiner Form gemischte Ester/Amide, höher alkylierte Diester und Diamide der Pyridin-2,4- und -2,5-dicarbonsäuren, die die Kollagenbiosynthese im Tiermodell wirksam hemmen. So wird in der DE-A 37 03 959 unter anderem die Synthese von N,N'-Bis(2-methoxyethyl)-pyridin-2,4-dicarbonsäurediamid und N,N'-Bis(3-isopropoxypropyl)-pyridin-2,4-dicarbonsäurediamid beschrieben.

In den deutschen Patentanmeldungen P 38 26 471.4 und P 38 28 140.6 wird ein verbessertes Verfahren zur Herstellung von N,N'-Bis(2-methoxyethyl)-pyridin-2,4-dicarbonsäurediamidvorgeschlagen.

Die deutsche Patentanmeldung P 39 24 093.2 schlägt neue N,N'-Bis(alkoxyalkyl)-pyridin-2,4-dicarbonsäurediamide vor.

Die deutsche Patentanmeldung P 40 01 002.3 beschreibt die Verwendung von Pyridin-2,4- und -2,5-dicarbonsäuredi(nitroxyalkyl)amide zur Herstellung von Prolin- und Lysinhydroxylase hemmenden Arzneimitteln.

Sowohl Pyridin-2,4- und -2,5-dicarbonsäurediamid (Hirakata et al., J. pharm. Soc. Japan 77 (1957) 219 und Häring et al., Helv. 37 (1954) 147, 153) als auch Pyridin-2,4 und -2,5-dicarbonsäuredihydrazid (Itai et al., Bl. nation. hyg. Labor. Tokyo, 74 (1956) 115, 117 und Shinohara et al., Chem. High Polymers Japan, 15 (1958) 839) sind bereits als Tuberkulosemittel bekannt.

In der JP 53/28175 (78/28175) werden N,N'-bis(2-nitrooxyethyl)pyridin-2,4- und -2,5-dicarbonsäurediamide als Substanzen mit vasodilatorischer Wirkung beschrieben.

Die deutsche Patentanmeldung P 40 20 570.3 beschreibt die Verwendung von 2,4- und 2,5-substituierten Pyridin-N-oxiden zur Herstellung von Prolin- und Lysinhydroxylase hemmenden Arzneimitteln.

Überraschend wurde nun gefunden, daß 4- oder 5-substituierte Pyridin-2-carbonsäuren der unten angegebenen allgemeinen Formel I sowie deren physiologisch verträglichen Salze die Lysin- und Prolinhydroxylase im Tiermodell wirksam inhibieren.

Erfindungsgemäß beansprucht werden daher Verbindungen gemäß Formel I

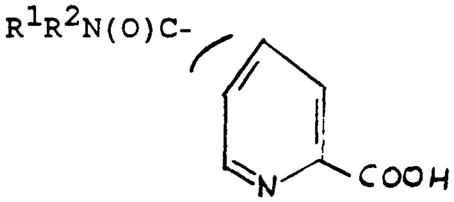

(I)

worin

$R^1$ und $R^2$ unabhängig voneinander Wasserstoff, $C_1$-$C_{12}$-Alkyl, $C_2$-$C_{12}$-Alkenyl, $C_2$-$C_{12}$-Alkinyl, nicht-

benzoanneliertes oder benzoanneliertes $C_5$-$C_7$-Cycloalkyl, Aryl oder Heteroaryl bedeuten, wobei diese für $R^1$ und $R^2$ genannten Reste unsubstituiert sind oder substituiert sind mit einem oder mehreren gleichen oder unterschiedlichen Resten $R^3$, wobei

$R^3$ Halogen, Hydroxy, Cyano, Nitro, Nitroxy, Amino, Carboxyl, $C_1$-$C_4$-Alkoxy, $C_1$-$C_4$-Alkoxycarbonyl, $C_1$-$C_4$-Alkyl- oder -dialkylamino, Indolyl oder Phenyl bedeutet, wobei der Indolyl- und Phenylrest unsubstituiert ist oder 1-, 2- oder 3-fach substituiert ist mit Halogen, Nitro, $C_1$-$C_4$-Alkyl oder $C_1$-$C_4$-Alkoxy, wobei bei Mehrfachsubstitution die Reste gleich oder verschieden sind

oder

$R^2$ sofern $R^1$ Wasserstoff bedeutet, einen Rest -N($R^4$)($R^5$) darstellt, worin

$R^4$ und $R^5$ gleich oder verschieden sind und Wasserstoff, $C_1$-$C_4$-Alkyl, $C_1$-$C_3$-Alkylcarbonyl oder Phenyl bedeuten

oder

$R^2$ sofern $R^1$ Wasserstoff bedeutet, einen Rest einer über N-Terminus gebundene $\alpha$-Aminosäure, $\alpha$-Aminosäurealkylester, $\alpha$-Aminosäureamid, $\alpha$-Aminosäurealkyl- oder -dialkylamid, wobei die genannten Alkylreste 1 bis 4 C-Atome aufweisen und gegebenenfalls mit Phenyl monosubstituiert sind und wobei die $C_3$- und $C_4$-Alkylreste auch verzweigt sein können,

oder

$R^2$ sofern $R^1$ Wasserstoff bedeutet, einen Rest über N-Terminus gebundenes Di- oder Tripeptid bedeutet, wobei als "Rest" die jeweilige $\alpha$-Aminosäure, und deren Derivate oder das Di- oder Tripeptid, jeweils abzüglich des N-Terminus bedeutet

und

$R^1$ die Bedeutung von $R^2$ hat, wobei die Reste $R^1$ und $R^2$ gleich oder verschieden sind oder

$R^1$ und $R^2$ zusammen mit dem Stickstoffatom, an dem sie gebunden sind, einen Rest der Formel II

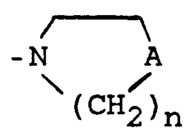

(II)

darstellen,

worin

n 1 bis 3 ist und

A O, S, $CH_2$ oder -N($R^6$)- bedeutet, wobei

$R^6$ Wasserstoff, Phenyl, $C_1$-$C_6$-Alkyl, $C_2$-$C_6$-Alkenyl oder $C_2$-$C_6$-Alkinyl bedeutet, wobei diese genannten Reste unsubstituiert sind oder substituiert sind mit Phenyl, welches seinerseits unsubstituiert ist oder ein- oder mehrfach substituiert ist mit einem oder mehreren gleichen oder verschiedenen Substituenten, ausgewählt aus: Halogen, Nitro, Cyano, Carboxy, Hydroxy, Methyl, Ethyl, Methoxy, Ethoxy und Trifluormethyl

oder

-N($R^7$)$_2$, wobei

$R^7$ Wasserstoff oder $C_1$-$C_3$-Alkyl bedeutet,

oder

-COOR$^7$

oder

-CON($R^8$)$_2$ oder CONHR$^6$, wobei

$R^8$ die Bedeutung von $R^7$ hat oder wobei ($R^8$)$_2$ eine $C_4$-$C_6$-Alkylenkette darstellt, worin keine oder eine $CH_2$-Gruppe, welche nicht direkt benachbart zu dem Stickstoffatom steht, ersetzt ist durch O, S oder N-$R^7$

oder wobei

$R^6$ $C_1$-$C_4$-Alkoxycarbonyl oder $C_3$-$C_7$-Cycloalkyl bedeutet,

sowie die physiologisch verträglichen Salze, wobei Pyridin-2-carbonsäure-4- und 5-carbonsäureamid ausgenommen sind.

Insbesondere bevorzugt sind Verbindungen der allgemeinen Formel I, worin

$R^1$ und $R^2$ unabhängig voneinander Wasserstoff, $C_1$-$C_6$-Alkyl, $C_2$-$C_6$-Alkenyl, $C_2$-$C_6$-Alkinyl, $C_5$-$C_7$-Cycloalkyl, Aryl oder Heteroaryl bedeutet, wobei diese für $R^1$ und $R^2$ genannten Reste unsubstituiert sind oder substituiert sind mit einem oder zwei gleichen oder unterschiedli-

chen Resten $R^3$, wobei

$R^3$ Halogen, Hydroxy, Cyano, Amino, Carboxyl, $C_1$-$C_4$-Alkoxy, $C_1$-$C_4$-Alkoxycarbonyl, $C_1$-$C_4$-Alkyl- oder -dialkylamino, oder Phenyl bedeutet, wobei der Phenylrest unsubstituiert ist oder 1-fach substituiert ist mit Halogen, $C_1$-$C_2$-Alkyl oder $C_1$-$C_2$-Alkoxy,

oder

$R^2$ sofern $R^1$ Wasserstoff bedeutet, einen Rest $-N(R^4)(R^5)$ darstellt worin $R^4$ und $R^5$ gleich oder verschieden sind und Wasserstoff oder $C_1$-$C_3$-Alkyl darstellen,

oder

$R^2$ sofern $R^1$ Wasserstoff bedeutet, einen Rest einer über N-Terminus gebundenen $\alpha$-Aminosäure oder $\alpha$-Aminosäurealkylester, wobei der Alkylrest 1 bis 3 C-Atome aufweist und gegebenenfalls mit Phenyl monosubstituiert ist und wobei der $C_3$-Alkylrest auch verzweigt sein kann,

und

$R^1$ die Bedeutung von $R^2$ hat, wobei die Reste $R^1$ und $R^2$ gleich oder verschieden sind oder

$R^1$ und $R^2$ zusammen mit dem Stickstoffatom, an dem sie gebunden sind, einen Rest der Formel II

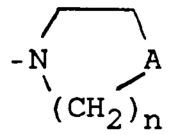

(II)

darstellen,

worin

n 1 bis 3 ist und

A O, $CH_2$ oder $-N(R^6)-$ bedeutet, wobei

$R^6$ Wasserstoff, Phenyl, $C_1$-$C_6$-Alkyl bedeutet, wobei diese genannten Reste unsubstituiert sind oder substituiert sind mit $C_1$-$C_4$-Alkoxycarbonyl oder $C_3$-$C_7$-Cycloalkyl

sowie die physiologisch verträglichen Salze, wobei Pyridin-2-carbonsäure-4 und 5-carbonsäureamid ausgenommen sind.

Besonders bevorzugt sind Verbindungen der allgemeinen Formel I, worin

$R^1$ und $R^2$ unabhängig voneinander Wasserstoff, $C_1$-$C_6$-Alkyl, insbesondere $C_1$-$C_3$-Alkyl, $C_6$-Cycloalkyl, Phenyl, oder Pyridyl bedeutet, wobei diese für $R^1$ und $R^2$ genannten Reste unsubstituiert sind oder substituiert sind mit einem oder zwei gleichen Resten $R^3$, wobei

$R^3$ Hydroxy, Amino, Carboxyl, $C_1$-$C_4$-Alkoxy, insbesondere Methoxy, $C_1$-$C_4$-Alkoxycarbonyl, oder Phenyl bedeutet, wobei der Phenylrest unsubstituiert ist oder 1-fach substituiert ist mit Methyl oder Methoxy

und

$R^1$ die Bedeutung von $R^2$ hat, wobei die Reste $R^1$ und $R^2$ gleich oder verschieden sind oder

$R^1$ und $R^2$ zusammen mit dem Stickstoffatom, an dem sie gebunden sind, einen Rest der Formel II

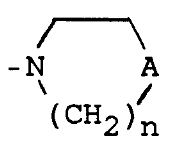

(II)

darstellen,

worin

n 2 ist und

A O oder $CH_2$ bedeutet,

sowie die physiologisch verträglichen Salze, wobei Pyridin-2-carbonsäure-4- und 5-carbonsäureamid ausgenommen sind.

Die genannten Verbindungen Pyridin-2-carbonsäure-4 und 5-carbonsäureamid sind als solche schon beschrieben in Thums, L. J. Pharm. Belg. 24 (1-2), 3-21 (1969) und Delarge, J.: Pharm. Acta. Helv. 44 (10), 637-43 (1969).

Unter Halogen werden Fluor, Chlor, Brom und Jod, unter Aryl Phenyl und Naphthyl und unter

Heteroaryl 5- und 6-gliedrige aromatische Ringe mit 1, 2 oder 3 Stickstoff- und/oder Sauerstoff- und/oder Schwefelatomen verstanden, die gegebenenfalls noch benzoanneliert sein können; insbesondere handelt es sich bei den Heteroarylresten um Pyridyl-, Pyridazyl-, Pyrimidyl-, Pyrazyl-, 1,3,5-Triazyl-, Pyrrolyl-, Pyrazolyl-, Imidazolyl-, Triazolyl-, Tetrazolyl-, Thienyl-, Oxazolyl- und Thiazolyl-Reste und gegebenenfalls deren benzoannelierte Verbindungen.

"Mehrfach substituiert" bedeutet im Vorstehenden und Folgenden, daß mindestens 2 höchstens alle in den Alkylresten vorhandenen Wasserstoffatome durch die genannten Substituenten ersetzt sind. Bevorzugt handelt es sich dabei um einen Substituenten pro Methyl- bzw. Methylengruppe.

Bei Mehrfachsubstitutionen können die Substituenten auch voneinander unabhängig sein.

Weiterhin betrifft die Erfindung die Verwendung von Verbindungen der allgemeinen Formel I sowie die physiologisch verträglichen Salze zuzüglich Pyridin-2-carbonsäure-4- und 5-carbonsäureamid zur Herstellung eines Prolin- und Lysinhydroxylase hemmenden Arzneimittels.

Schließlich betrifft die Erfindung die Verbindungen der allgemeinen Formel I zuzüglich Pyridin-2-carbonsäure-4- und 5-carbonsäureamid zur Verwendung als Arzneimittel.

Insbesondere betrifft die Erfindung die Verbindungen der Formel I zuzüglich Pyridin-2-carbonsäure-4- und 5-carbonsäureamid zur Anwendung als Fibrosuppressiva und Immunsuppressiva sowie zur Inhibierung der Prolin- und Lysinhydroxylase und zur Beeinflussung des Stoffwechsels von Kollagen und kollagenähnlichen Stoffen bzw. der Biosynthese von C1q.

Alle genannten Alkylreste mit mehr als 2 C-Atomen können sowohl geradkettig als auch verzweigt sein.

Weiterhin betrifft die Erfindung ein Verfahren zur Herstellung von Verbindungen der allgemeinen Formel I zuzüglich Pyridin-2-carbonsäure-4- oder 5-carbonsäureamide.

Das erfindungsgemäße Verfahren wird im folgenden am Beispiel der Pyridin-2,4-dicarbonsäure verdeutlicht. Das Verfahren von erfindungsgemäßen Verbindungen mit 2,5-Substitution erfolgt in analoger Weise.

**Reaktionsschema**

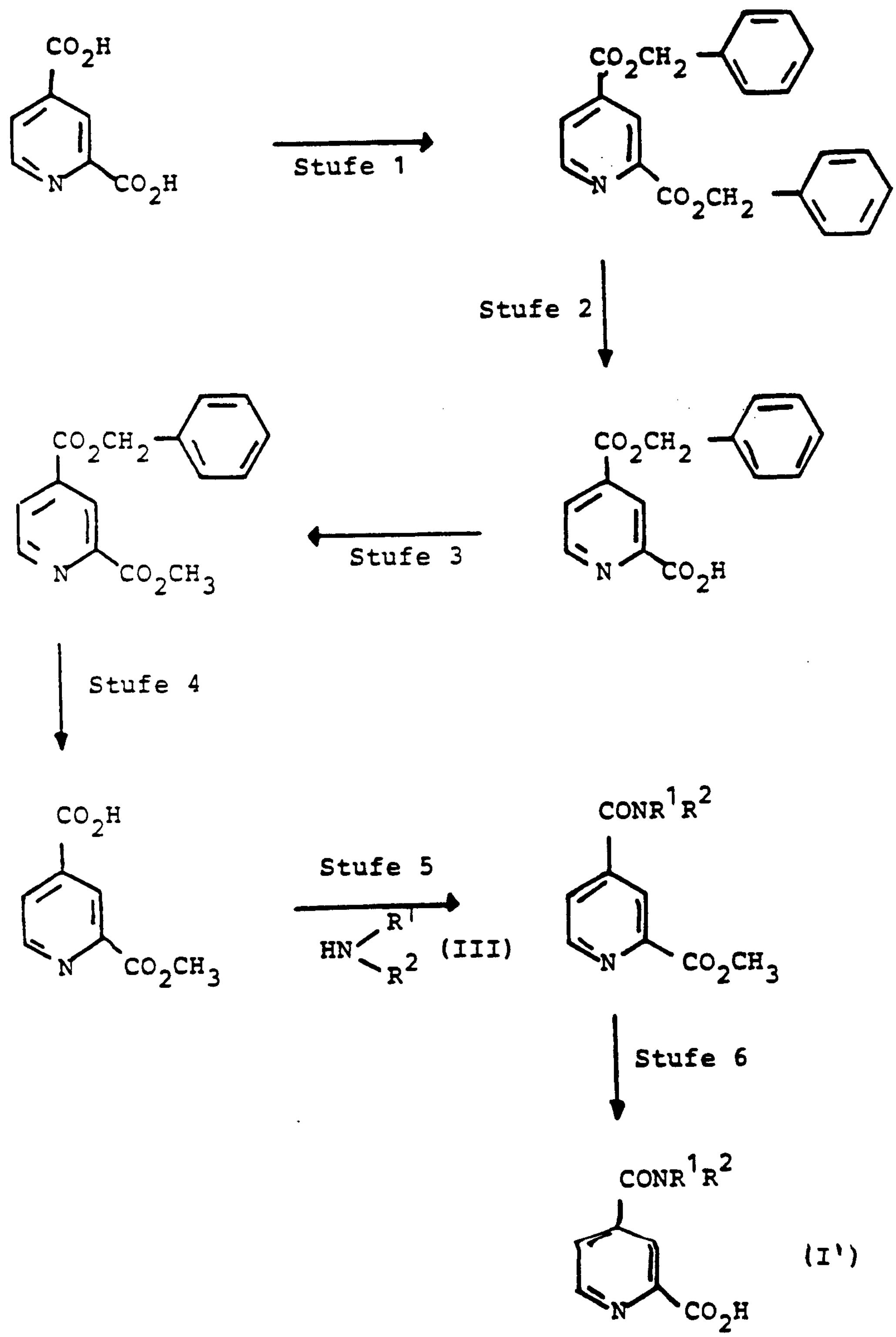


Gemäß Stufe 1 wird die käufliche Pyridin-2,4-dicarbonsäure in ihr Dicarbonsäuredihalogenid, vorzugsweise ihr -dichlorid, überführt und mit einem gegebenenfalls substituierten Benzylalkohol zum Pyridin-2,4-dicarbonsäuredibenzylester umgesetzt.

Gemäß Stufe 2 wird der Diester selektiv in 2-Stellung z.B. in Gegenwart eines Kupfer-Katalysators gemäß Delarge, J.: Phar. Acta. Helv. 44 (10), 637 (1969) verseift.

Die freie Säurefunktion in 2-Stellung wird anschließend in Stufe 3 in das entsprechende Säurechlorid überführt und mit einem Alkohol, wie z.B. Methyl- oder Ethylalkohol in den entsprechenden 2-Carbonsäureester umgesetzt.

Die verbliebene Benzylschutzgruppe in 4-Stellung wird gemäß Stufe 4 hydrogenolytisch abgespalten (z.B. mit $H_2$/Pd, Houben-Weyl: Bd. IV/1c (1980), S. 381-82) und die freie Säure in 4-Stellung in ihr Säurechlorid überführt.

Das Säurechlorid kann nun mit dem Amin der allgemeinen Formel III, in der $R^1$ und $R^2$ die Bedeutung wie in den Verbindungen der allgemeinen Formel I haben, in den gemischten Pyridin-4-carbonsäureamid-2-carbonsäureester überführt werden.

Der 2-Carbonsäureester kann nun abschließend (Stufe 6) mit alkoholischer Alkalilauge (z.B. Natronlauge in Ethanol) zunächst in das Salz der 2-Carbonsäure überführt werden, welches dann mit Mineralsäuren (wie z.B. Salzsäure) in die erfindungsgemäße Verbindung der Formel I' umgesetzt wird.

Das genannte Verfahren, das gemäß dem Reaktionsschema für die in 4-Position substituierten Verbindungen beschrieben worden ist, gilt auch für die Verbindungen, die in 5-Position entsprechend substituiert sind.

Die Verbindungen der allgemeinen Formel I zuzüglich Pyridin-2-carbonsäure-4- oder 5-carbonsäureamid besitzen wertvolle pharmakologische Eigenschaften und zeigen insbesondere Wirksamkeit als Hemmer der Prolin- und Lysinhydroxylase, als Fibrosuppressivum und Immunsuppressivum.

Die Aktivität der Fibrogenase kann durch radioimmunologische Bestimmung des N-terminalen Propeptids des Kollagens Typ-III oder der N- bzw. C-terminalen Quervernetzungsdomäne des Kollagens-Typ-IV (7s-Kollagen bzw. Typ-IV-Kollagen-$NC_1$) im Serum bestimmt werden.

Zu diesem Zweck wurden die Hydroxyprolin-, Prokollagen-III-Peptid-, 7s-Kollagen- und Typ-IV-Kollagen-$NC_1$-Konzentrationen in der Leber von

a) unbehandelten Ratten (Kontrolle)

b) Ratten, denen Tetrachlorkohlenstoff verabreicht wurde ($CCl_4$)-Kontrolle)

c) Ratten, denen zunächst $CCl_4$ und anschließend eine erfindungsgemäße Verbindung verabreicht wurde gemessen (diese Testmethode wird beschrieben von Rouiller, C., experimental toxic injury of the liver; in The Liver, C. Rouiller, Vol. 2, S. 335-476, New York, Academic Press, 1964).

Die Verbindungen der Formel I zuzüglich von Pyridin-2-carbonsäure-4- und 5-carbonsäureamid können als Medikamente in Form pharmazeutischer Präparate Verwendung finden, welche sie gegebenenfalls zusammen mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organische oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,01 - 25,0 mg/kg/Tag, vorzugsweise 0,01 - 5,0 mg/kg/Tag oder parenteral in Dosen von 0,001 - 5 mg/kg/Tag, vorzugsweise 0,001 - 2,5 mg/kg/Tag, insbesondere 0,005 - 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Stoffwechselstörungen eingesetzt werden.

Ein weiterer Gegenstand der Erfindung sind Arzneimittel, die ein oder mehrere erfindungsgemäße Verbindungen der Formel I und/oder deren physiologisch verträgliche Salze enthalten, zuzüglich der Pyridin-2-carbonsäure-4- und 5-carbonsäureamide.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die Wirkstoffe können oral, parenteral oder rektal appliziert werden.

Die aktiven Verbindungen werden mit den dafür geeigneten Zustzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige, alkoholische oder ölige Suspensionen oder wäßrige oder ölige Lösungen.

Als inerte Trägerstoffe können z.B. gummi arabicum, Magnesia, Magnesiumcarbonat, Kaliumphosphat, Milchzucker, Glukose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z.B. in Frage physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Nachfolgend ist die Erfindung anhand von Beispielen näher erläutert.

Beispiel 1

Pyridin-2,4-dicarbonsäuredibenzylester (Stufe 1)

30 g Pyridin-2,4-dicarbonsäure werden mit
30 ml Thionylchlorid ins Säurechlorid überführt und mit 43,8 g Benzylalkohol umgesetzt. Das Produkt wird aus Diisopropylether umkristallisiert.
Ausbeute: 42,1 g    Schmelzpunkt 63 - 65 °C

Beispiel 2

Pyridin-2-carbonsäure-4-carbonsäurebenzylester (Stufe 2)

40 g Pyridin-2,4-dicarbonsäuredibenzylester aus Beispiel 1 werden zu einer Suspension aus 27,8 g Kupfer-II-nitrat in 700 ml Methanol gegeben. Es wird eine Stunde unter Rückfluß gekocht und nach dem Abkühlen vom Kupferkomplex abfiltriert. Der Komplex wird in Dioxan suspendiert und Schwefelwasserstoff eingeleitet. Das ausgefallene Kupfersulfid wird abfiltriert und die organische Phase eingeengt. Das Produkt wird mit Petrolether verrührt.
Ausbeute: 25,3 g    Schmelzpunkt 113 - 115 °C

Beispiel 3

Pyridin-4-carbonsäurebenzylester-2-carbonsäuremethylester (Stufe 3)

34 g der Verbindung aus Beispiel 2 werden in 1 l Tetrahydrofuran vorgelegt. Man kühlt auf 0 °C ab und tropft bei dieser Temperatur 250 ml einer Diazomethan-Lösung langsam zu. Man läßt auf Raumtemperatur kommen und 12 Stunden rühren. Die Lösung wird eingeengt und das Rohprodukt chromatographiert (Kieselgel, Laufmittel Ethylacetat).
Ausbeute: 26 g    Schmelzpunkt: 58 °C

Beispiel 4

Pyridin-4-carbonsäure-2-carbonsäuremethylester (Stufe 4)

Bei Raumtemperatur werden 26 g der Verbindung aus Beispiel 3 in 1 l Dioxan gelöst. Zur Lösung gibt man 500 mg Palladium/Kohle (10 %)-Katalysator und hydriert 4 Stunden unter Normaldruck. Nach Beendigung der Wasserstoffaufnahme wird vom Katalysator abgesaugt und vom Lösungsmittel abgezogen.
Ausbeute: 15,3 g    Schmelzpunkt: 241-243 °C

Beispiel 5a

Pyridin-2-carbonsäuremethylester-4-carbonsäure-(2-methoxyethyl)-amid (Stufe 5)

11 g der Verbindung aus Beispiel 4 werden analog Beispiel 1 mit 4,8 ml Thionylchlorid in das Säurechlorid überführt und mit 5,7 ml 2-Methoxyethylamin zum Amid umgesetzt. Das Rohprodukt wird mit

Diisopropylether verrieben und abgesaugt.
Ausbeute: 9,5 g  Schmelzpunkt: 74 - 76 ° C

Beispiel 5b

Pyridin-2-carbonsäuremethylester-4-carbonsäure-(3-methoxypropyl)-amid (Stufe 5)

Analog Beispiel 5a werden 3 g der Verbindung aus Beispiel 4 mit 1,2 ml Thionylchlorid in das Säurechlorid überführt und mit 1,7 ml 3-Methoxypropylamin zum Amid umgesetzt. Das Rohprodukt wird chromatographiert (Ethylacetat/Methanol 15/1).
Ausbeute: 2,6 g  Öl

Beispiel 6a

Pyridin-2-carbonsäure-4-carbonsäure-(2-methoxyethyl)-amid (Stufe 6)

Bei Raumtemperatur werden 2,3 g Natronlauge (Plätzchen) in 70 ml Ethanol gelöst. Bei dieser Temperatur wird eine Lösung aus 9 g der Verbindung aus Beispiel 5a in 30 ml Ethanol zugetropft. Nach 4 Stunden wird zur Trockne eingedampft, mit wenig Wasser aufgenommen und langsam mit konz. Salzsäure angesäuert. Die Lösung wird eingedampft und das Produkt aus heißem Ethanol umkristallisiert.
Ausbeute: 8 g  Schmelzpunkt: 130 - 135 ° C

Beispiel 6b

Pyridin-2-carbonsäure-4-carbonsäure-(3-methoxypropyl)-amid (Stufe 6)

Die Verbindung wird analog zu Beispiel 6a aus 3 g des Esters aus Beispiel 5b hergestellt.
Ausbeute: 2,6 g  Schmelzpunkt: 118 - 122 ° C

**Patentansprüche**

**1.** 4- oder 5-substituierte Pyridin-2-carbonsäuren der Formel I

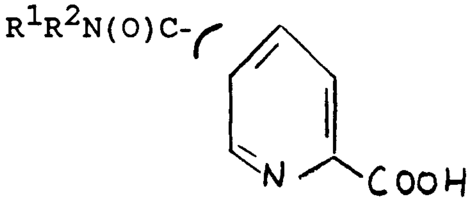

(I)

worin

$R^1$ und $R^2$ unabhängig voneinander Wasserstoff, $C_1$-$C_{12}$-Alkyl, $C_2$-$C_{12}$-Alkenyl, $C_2$-$C_{12}$-Alkinyl, nichtbenzoanneliertes oder benzoanneliertes $C_5$-$C_7$-Cycloalkyl, Aryl oder Heteroaryl bedeutet, wobei diese für $R^1$ und $R^2$ genannten Reste unsubstituiert sind oder substituiert sind mit einem oder mehreren gleichen oder unterschiedlichen Resten $R^3$, wobei

$R^3$ Halogen, Hydroxy, Cyano, Nitro, Nitroxy, Amino, Carboxyl, $C_1$-$C_4$-Alkoxy, $C_1$-$C_4$-Alkoxycarbonyl, $C_1$-$C_4$-Alkyl- oder -dialkylamino, Indolyl oder Phenyl bedeutet, wobei der Indolyl- und Phenylrest unsubstituiert ist oder 1-, 2- oder 3-fach substituiert ist mit Halogen, Nitro, $C_1$-$C_4$-Alkyl oder $C_1$-$C_4$-Alkoxy, wobei bei Mehrfachsubstitution die Reste gleich oder verschieden sind
oder

$R^2$ sofern $R^1$ Wasserstoff bedeutet, einen Rest
$-N(R^4)(R^5)$ darstellt, worin

$R^4$ und $R^5$ gleich oder verschieden sind und Wasserstoff, $C_1$-$C_4$-Alkyl, $C_1$-$C_3$-Alkylcarbonyl oder Phenyl bedeuten

oder

$R^2$ sofern $R^1$ Wasserstoff bedeutet, einen Rest einer über N-Terminus gebundene $\alpha$-Aminosäure, $\alpha$-Aminosäurealkylester, $\alpha$-Aminosäureamid, $\alpha$-Aminosäurealkyl- oder -dialkylamid, wobei die genannten Alkylreste 1 bis 4 C-Atome aufweisen und gegebenenfalls mit Phenyl monosubstituiert sind und wobei die $C_3$- und $C_4$-Alkylreste auch verzweigt sein können,

oder

$R^2$ sofern $R^1$ Wasserstoff bedeutet, einen Rest über N-Terminus gebundenes Di- oder Tripeptid bedeutet, wobei als "Rest" die jeweilige $\alpha$-Aminosäure, und deren Derivate oder das Di- oder Tripeptid, jeweils abzüglich des N-Terminus bedeutet

und

$R^1$ die Bedeutung von $R^2$ hat, wobei die Reste $R^1$ und $R^2$ gleich oder verschieden sind

oder

$R^1$ und $R^2$ zusammen mit dem Stickstoffatom, an dem sie gebunden sind, einen Rest der Formel II

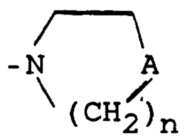

(II)

darstellen,

worin

n 1 bis 3 ist und

A O, S, $CH_2$ oder $-N(R^6)-$ bedeutet, wobei

$R^6$ Wasserstoff, Phenyl, $C_1$-$C_6$-Alkyl, $C_2$-$C_6$-Alkenyl oder $C_2$-$C_6$-Alkinyl bedeutet, wobei diese genannten Reste unsubstituiert sind oder substituiert sind mit Phenyl, welches seinerseits unsubstituiert ist oder ein oder mehrfach substituiert ist mit einem oder mehreren gleichen oder verschiedenen Substituenten, ausgewählt aus: Halogen, Nitro, Cyano, Carboxy, Hydroxy, Methyl, Ethyl, Methoxy, Ethoxy und Trifluormethyl

oder

$-N(R^7)_2$, wobei

$R^7$ Wasserstoff oder $C_1$-$C_3$-Alkyl bedeutet,

oder

$-COOR^7$

oder

$-CON(R^8)_2$ oder $CONHR^6$, wobei

$R^8$ die Bedeutung von $R^7$ hat oder wobei $(R^8)_2$ eine $C_4$-$C_6$-Alkylenkette darstellt, worin keine oder eine $CH_2$-Gruppe, welche nicht direkt benachbart zu dem Stickstoffatom steht, ersetzt ist durch O, S oder $N-R^7$

oder wobei

$R^6$ $C_1$-$C_4$-Alkoxycarbonyl oder $C_3$-$C_7$-Cycloalkyl bedeute,

sowie die physiologisch verträglichen Salze, wobei Pyridin-2-carbonsäure-4- und 5-carbonsäureamid ausgenommen sind.

**2.** Verbindungen nach Anspruch 1, worin

$R^1$ und $R^2$ unabhängig voneinander Wasserstoff, $C_1$-$C_6$-Alkyl, $C_2$-$C_6$-Alkenyl, $C_2$-$C_6$-Alkinyl, $C_5$-$C_7$-Cycloalkyl, Aryl oder Heteroaryl bedeutet, wobei diese für $R^1$ und $R^2$ genannten Reste unsubstituiert sind oder substituiert sind mit einem oder zwei gleichen oder unterschiedlichen Resten $R^3$, wobei

$R^3$ Halogen, Hydroxy, Cyano, Amino, Carboxyl, $C_1$-$C_4$-Alkoxy, $C_1$-$C_4$-Alkoxycarbonyl, $C_1$-$C_4$-Alkyl- oder -dialkylamino, oder Phenyl bedeutet, wobei der Phenylrest unsubstituiert ist oder 1-fach substituiert ist mit Halogen, $C_1$-$C_2$-Alkyl oder $C_1$-$C_2$-Alkoxy,

oder

$R^2$ sofern $R^1$ Wasserstoff bedeutet, einen Rest $-N(R^4)(R^5)$ darstellt worin $R^4$ und $R^5$ gleich oder verschieden sind und Wasserstoff oder $C_1$-$C_3$-Alkyl darstellen,

oder

$R^2$ sofern $R^1$ Wasserstoff bedeutet, einen Rest einer über N-Terminus gebundenen a-Aminosäure oder a-Aminosäurealkylester, wobei der Alkylrest 1 bis 3 C-Atome aufweist und gegebenenfalls mit Phenyl monosubstituiert sind und wobei der $C_3$-Alkylrest auch verzweigt sein kann,

und

$R^1$ die Bedeutung von $R^2$ hat, wobei die Reste $R^1$ und $R^2$ gleich oder verschieden sind oder

$R^1$ und $R^2$ zusammen mit dem Stickstoffatom, an dem sie gebunden sind, einen Rest der Formel II

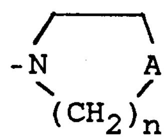

(II)

darstellen,

worin

n 1 bis 3 ist und

A O, $CH_2$ oder $-N(R^6)-$ bedeuten, wobei

$R^6$ Wasserstoff, Phenyl, $C_1$-$C_6$-Alkyl bedeutet, wobei diese genannten Reste unsubstituiert sind oder substituiert sind mit

$C_1$-$C_4$-Alkoxycarbonyl oder $C_3$-$C_7$-Cycloalkyl

sowie die physiologisch verträglichen Salze, wobei Pyridin-2-carbonsäure-4 und 5-carbonsäureamid ausgenommen sind.

**3.** Verbindungen nach Anspruch 1, worin

$R^1$ und $R^2$ unabhängig voneinander Wasserstoff, $C_1$-$C_5$-Alkyl, insbesondere $C_1$-$C_3$-Alkyl, $C_6$-Cycloalkyl, Phenyl, oder Pyridyl bedeutet, wobei diese für $R^1$ und $R^2$ genannten Reste unsubstituiert sind oder substituiert sind mit einem oder zwei gleichen Resten $R^3$, wobei

$R^3$ Hydroxy, Amino, Carboxyl, $C_1$-$C_4$-Alkoxy, insbesondere Methoxy, $C_1$-$C_4$-Alkoxycarbonyl, oder Phenyl bedeutet, wobei der Phenylrest unsubstituiert ist oder 1-fach substituiert ist mit Methyl oder Methoxy

und

$R^1$ die Bedeutung von $R^2$ hat, wobei die Reste $R^1$ und $R^2$ gleich oder verschieden sind oder

$R^1$ und $R^2$ zusammen mit dem Stickstoffatom, an dem sie gebunden sind, einen Rest der Formel II

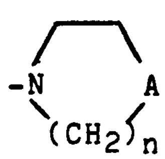

(II)

darstellen,

worin

n 2 ist und

A O oder $CH_2$ bedeutet,

sowie die physiologisch verträglichen Salze, wobei Pyridin-2-carbonsäure-4 und 5-carbonsäureamid ausgenommen sind.

**4.** Verfahren zur Herstellung von 4- oder 5-substituierten Pyridin-2-carbonsäuren der Formel I sowie deren physiologisch verträglichen Salze zuzüglich von Pyridin-2-carbonsäure-4- und 5-carbonsäureamide, wobei man

- Pyridin-2,4-dicarbonsäure in ihr Dihalogenid,

- das Dihalogenid in den Dibenzylester überführt,
- den Dibenzylester selektiv in 2-Stellung verseift,
- den den daraus entstehenden Mono-benzylester an der freien Säurefunktion in das entsprechende Säurechlorid überführt und mit einem Alkohol, der nicht Benzylalkohol entspricht, in den entsprechenden 2-Carbonsäureester umsetzt,
- die Benzylschutzgruppe in 4- bzw. 5-Stellung hydrogenolytisch abspaltet und die entstandene freie Säure in 4- bzw. 5-Stellung in das entsprechende Säurechlorid überführt, dadurch gekennzeichnet, daß
- das Säurechlorid der vorherigen Stufe mit einem Amin der allgemeinen Formel III

  $HNR^1R^2$ (III)

  wobei $R^1$ und $R^2$ eine in Formel I angegebene Bedeutung aufweisen, in den gemischten Pyridindicarbonsäure-4 oder 5-amid-2-ester überführt wird und
- abschließend den genannten 2-Carbonsäureester mit Mineralsäure in die in 2-Stellung freie Säure überführt.

**5.** Verbindungen nach Anspruch 4 zur Inhibierung der Prolin- und Lysinhydroxylase.

**6.** Verbindungen nach Anspruch 4 zur Anwendung als Fibrosuppressiva und Immunsuppressiva.

**7.** Arzneimittel, enthalten eine Verbindung nach Anspruch 4 und/oder deren physiologisch verträglichen Salze zur Behandlung von Störungen der Biosynthese von Kollagen und kollagenähnlichen Stoffen bzw. der Biosynthese von $C1_q$.

**8.** Verfahren zur Herstellung von Arzneimitteln zur Beeinflussung der Biosynthese von Kollagen und kollagenähnlichen Stoffen bzw. der Biosynthese von $C1_q$,dadurch gekennzeichnet, daß man in das Arzneimittel eine Verbindung der Formel I nach Anspruch 4 und/oder ein physiologisch verträgliches Salz dieser Verbindung einverleibt.

**Patentansprüche für folgenden Vertragsstaat : ES**

**1.** Verfahren zur Herstellung von 4- oder 5-substituierten Pyridin-2-carbonsäuren der Formel I

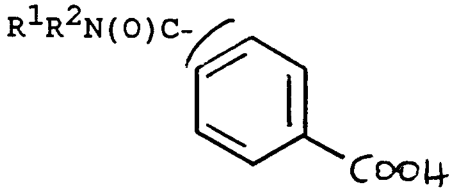

(I)

worin

$R^1$ und $R^2$ unabhängig voneinander Wasserstoff, $C_1$-$C_{12}$-Alkyl, $C_2$-$C_{12}$-Alkenyl, $C_2$-$C_{12}$-Alkinyl, nichtbenzoanneliertes oder benzoanneliertes $C_5$-$C_7$-Cycloalkyl, Aryl oder Heteroaryl bedeutet, wobei diese für $R^1$ und $R^2$ genannten Reste unsubstituiert sind oder substituiert sind mit einem oder mehreren gleichen oder unterschiedlichen Resten $R^3$, wobei

$R^3$ Halogen, Hydroxy, Cyano, Nitro, Nitroxy, Amino, Carboxyl, $C_1$-$C_4$-Alkoxy, $C_1$-$C_4$-Alkoxycarbonyl, $C_1$-$C_4$-Alkyl- oder -dialkylamino, Indolyl oder Phenyl bedeutet, wobei der Indolyl- und Phenylrest unsubstituiert ist oder 1-, 2- oder 3-fach substituiert ist mit Halogen, Nitro, $C_1$-$C_4$-Alkyl oder $C_1$-$C_4$-Alkoxy, wobei bei Mehrfachsubstitution die Reste gleich oder verschieden sind

oder

$R^2$ sofern $R^1$ Wasserstoff bedeutet, einen Rest -N($R^4$)($R^5$) darstellt, worin

$R^4$ und $R^5$ gleich oder verschieden sind und Wasserstoff, $C_1$-$C_4$-Alkyl, $C_1$-$C_3$-Alkylcarbonyl oder Phenyl bedeuten

oder

$R^2$ sofern $R^1$ Wasserstoff bedeutet, einen Rest einer über N-Terminus gebundene $\alpha$-Aminosäure, $\alpha$-Aminosäurealkylester, $\alpha$-Aminosäureamid, $\alpha$-Aminosäurealkyl- oder -dialkylamid, wobei die genannten Alkylreste 1 bis 4 C-Atome aufweisen und gegebenenfalls mit Phenyl monosubstituiert sind und wobei die $C_3$- und $C_4$-Alkylreste auch verzweigt sein können,

oder

$R^2$ sofern $R^1$ Wasserstoff bedeutet, einen Rest über N-Terminus gebundenes Di- oder Tripeptid bedeutet, wobei als "Rest" die jeweilige $\alpha$-Aminosäure, und deren Derivate oder das Di- oder Tripeptid, jeweils abzüglich des N-Terminus bedeutet

und

$R^1$ die Bedeutung von $R^2$ hat, wobei die Reste $R^1$ und $R^2$ gleich oder verschieden sind

oder

$R^1$ und $R^2$ zusammen mit dem Stickstoffatom, an dem sie gebunden sind, einen Rest der Formel II

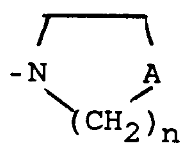

(II)

darstellen,

worin

n 1 bis 3 ist und

A O, S, $CH_2$ oder -N($R^6$)- bedeutet, wobei

$R^6$ Wasserstoff, Phenyl, $C_1$-$C_6$-Alkyl, $C_2$-$C_6$-Alkenyl oder $C_2$-$C_6$-Alkinyl bedeutet, wobei diese genannten Reste unsubstituiert sind oder substituiert sind mit Phenyl, welches seinerseits unsubstituiert ist oder ein oder mehrfach substituiert ist mit einem oder mehreren gleichen oder verschiedenen Substituenten, ausgewählt aus: Halogen, Nitro, Cyano, Carboxy, Hydroxy, Methyl, Ethyl, Methoxy, Ethoxy und Trifluormethyl

oder

-N($R^7$)$_2$, wobei

$R^7$ Wasserstoff oder $C_1$-$C_3$-Alkyl bedeutet,

oder

-COOR$^7$

oder

-CON($R^8$)$_2$ oder CONHR$^6$, wobei

$R^8$ die Bedeutung von $R^7$ hat oder wobei ($R^8$)$_2$ eine $C_4$-$C_6$-Alkylenkette darstellt, worin keine oder eine $CH_2$-Gruppe, welche nicht direkt benachbart zu dem Stickstoffatom steht, ersetzt ist durch O, S oder N-$R^7$

oder wobei

$R^6$ $C_1$-$C_4$-Alkoxycarbonyl oder $C_3$-$C_7$-Cycloalkyl bedeutet,

sowie die physiologisch verträglichen Salze, dadurch gekennzeichnet, daß man

- Pyridin-2,4-dicarbonsäure in ihr Dihalogenid,
- das Dihalogenid in den Dibenzylester überführt,
- den Dibenzylester selektiv in 2-Stellung verseift,
- den den daraus entstehenden Mono-benzylester an der freien Säurefunktion in das entsprechende Säurechlorid überführt und mit einem Alkohol, der nicht Benzylalkohol entspricht, in den entsprechenden 2-Carbonsäureester umsetzt,
- die Benzylschutzgruppe in 4- bzw. 5-Stellung hydrogenolytisch abspaltet und die entstandene freie Säure in 4- bzw. 5-Stellung in das entsprechende Säurechlorid überführt, dadurch gekennzeichnet, daß
- das Säurechlorid der vorherigen Stufe mit einem Amin der allgemeinen Formel III

$HNR^1R^2$ (III)

wobei $R^1$ und $R^2$ eine in Formel I angegebene Bedeutung aufweisen, in den gemischten Pyridindicarbonsäure-4 oder 5-amid-2-ester überführt wird und

- abschließend den genannten 2-Carbonsäureester mit Mineralsäure in die in 2-Stellung freie Säure überführt.

**2.** Verfahren nach Anspruch 1, worin

$R^1$ und $R^2$ unabhängig voneinander Wasserstoff, $C_1$-$C_6$-Alkyl, $C_2$-$C_6$-Alkenyl, $C_2$-$C_6$-Alkinyl, $C_5$-$C_7$-Cycloalkyl, Aryl oder Heteroaryl bedeutet, wobei diese für $R^1$ und $R^2$ genannten Reste unsubstituiert sind oder substituiert sind mit einem oder zwei gleichen oder unterschiedlichen Resten $R^3$, wobei

$R^3$ Halogen, Hydroxy, Cyano, Amino, Carboxyl, $C_1$-$C_4$-Alkoxy, $C_1$-$C_4$-Alkoxycarbonyl, $C_1$-$C_4$-Alkyl- oder -dialkylamino, oder Phenyl bedeutet, wobei der Phenylrest unsubstituiert ist oder 1-fach substituiert ist mit Halogen, $C_1$-$C_2$-Alkyl oder $C_1$-$C_2$-Alkoxy, oder

$R^2$ sofern $R^1$ Wasserstoff bedeutet, einen Rest $-N(R^4)(R^5)$ darstellt worin $R^4$ und $R^5$ gleich oder verschieden sind und Wasserstoff oder $C_1$-$C_3$-Alkyl darstellen, oder

$R^2$ sofern $R^1$ Wasserstoff bedeutet, einen Rest einer über N-Terminus gebundenen $\alpha$-Aminosäure oder $\alpha$-Aminosäurealkylester, wobei der Alkylrest 1 bis 3 C-Atome aufweist und gegebenenfalls mit Phenyl monosubstituiert sind und wobei der $C_3$-Alkylrest auch verzweigt sein kann, und

$R^1$ die Bedeutung von $R^2$ hat, wobei die Reste $R^1$ und $R^2$ gleich oder verschieden sind oder

$R^1$ und $R^2$ zusammen mit dem Stickstoffatom, an dem sie gebunden sind, einen Rest der Formel II

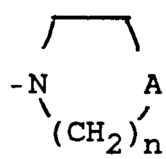

(II)

darstellen, worin

n 1 bis 3 ist und

A O, $CH_2$ oder $-N(R^6)-$ bedeuten, wobei

$R^6$ Wasserstoff, Phenyl, $C_1$-$C_6$-Alkyl bedeutet, wobei diese genannten Reste unsubstituiert sind oder substituiert sind mit $C_1$-$C_4$-Alkoxycarbonyl oder $C_3$-$C_7$-Cycloalkyl

sowie die physiologisch verträglichen Salze.

**3.** Verfahren nach Anspruch 1, worin

$R^1$ und $R^2$ unabhängig voneinander Wasserstoff, $C_1$-$C_5$-Alkyl, insbesondere $C_1$-$C_3$-Alkyl, $C_6$-Cycloalkyl, Phenyl, oder Pyridyl bedeutet, wobei diese für $R^1$ und $R^2$ genannten Reste unsubstituiert sind oder substituiert sind mit einem oder zwei gleichen Resten $R^3$, wobei

$R^3$ Hydroxy, Amino, Carboxyl, $C_1$-$C_4$-Alkoxy, insbesondere Methoxy, $C_1$-$C_4$-Alkoxycarbonyl, oder Phenyl bedeutet, wobei der Phenylrest unsubstituiert ist oder 1-fach substituiert ist mit Methyl oder Methoxy und

$R^1$ die Bedeutung von $R^2$ hat, wobei die Reste $R^1$ und $R^2$ gleich oder verschieden sind oder

$R^1$ und $R^2$ zusammen mit dem Stickstoffatom, an dem sie gebunden sind, einen Rest der Formel II

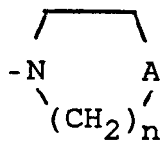

(II)

darstellen,
worin

n 2 ist und

A O oder $CH_2$ bedeutet,

sowie die physiologisch verträglichen Salze.

4. Verbindungen nach Anspruch 1 zur Inhibierung der Prolin- und Lysinhydroxylase.

5. Verbindungen nach Anspruch 1 zur Anwendung als Fibrosuppressiva und Immunsuppressiva.

6. Arzneimittel, enthalten eine Verbindung nach Anspruch 1 und/oder deren physiologisch verträglichen Salze zur Behandlung von Störungen der Biosynthese von Kollagen und kollagenähnlichen Stoffen bzw. der Biosynthese von $C1_q$.

7. Verfahren zur Herstellung von Arzneimitteln zur Beeinflussung der Biosynthese von Kollagen und kollagenähnlichen Stoffen bzw. der Biosynthese von $C1_q$,dadurch gekennzeichnet, daß man in das Arzneimittel eine Verbindung der Formel I nach Anspruch 1 und/oder ein physiologisch verträgliches Salz dieser Verbindung einverleibt.